# EUROPEAN PATENT APPLICATION

(11) **EP 2 587 396 A2**
(43) Date of publication of application: **01.05.2013**
(21) Application number: 12190557.4
(22) Date of filing: 30.10.2012
(51) Int. Cl.: G06F 19/22

(54) **Apparatus and method for generating novel sequence in target genome sequence**

(30) Priority: 31.10.2011 KR 20110112371
(71) Applicant: Samsung SDS Co., Ltd., Seoul 135-918 (KR)
(72) Inventor: Hong, Yoo Jin, 135-953 Seoul (KR); Lee, Yong Seok, 130-762 Seoul (KR); Shin, Soo Yong, 151-050 Seoul (KR)
(74) Representative: Grünecker, Kinkeldey, Stockmair & Schwanhäusser

(57) **Abstract**

An apparatus and method for generating a novel sequence in a target genome sequence for generating a novel sequence that does not exist in a reference sequence by using input reads that are not mapped to the reference sequence during genome re-sequencing of a next generation sequencing (NGS) technology. According to the present invention, the novel sequence that is not reflected to the reference sequence of the target genome sequence is generated, and information regarding the novel sequence may be provided.

## Description

### CROSS-REFERENCE TO RELATED PATENT APPLICATION

This application claims the benefit of Korean Patent Application No. 10-2011-0112371, filed on October 31, 2011, in the Korean Intellectual Property Office, the disclosure of which is incorporated herein in its entirety by reference.

### BACKGROUND OF THE INVENTION

### 1. Field of the Invention

The present invention relates to an apparatus and method for generating a novel sequence in a target genome sequence, and more particularly, to an apparatus and method for generating a novel sequence in a target genome sequence for generating a novel sequence that does not exist in a reference sequence by using input reads that are not mapped to the reference sequence during genome re-sequencing of a next generation sequencing (NGS) technology.

### 2. Description of the Related Art

An NGS technology produces a large amount of reads, which are short reads, when sequencing a target genome. The produced reads are mapped to a reference sequence, and a base sequence for the target genome is reconstituted with a consensus sequence of the mapped reads, and this process is referred to as re-sequencing. Thus, an individual genome sequence generated through re-sequencing is made based on a reference sequence.

As such, at present, NGS data constitutes a target genome sequence with a consensus sequence of reads mapped to a reference sequence.

However, due to a methodical limitation of re-sequencing, in an individual genome sequence that does not exist in a reference sequence or is different from the reference sequence, reads generated in the corresponding sequence may not be mapped to the reference sequence, and thus individual genetic characteristics may not be fully reflected in the individual genome sequence reconstituted according to a result of the re-sequencing. Accordingly, in order to obtain information regarding individual genetic characteristics to be differentiated from the reference sequence, although an additional analysis for the reads that are not mapped during the re-sequencing is required, the reads are generally excluded from the analysis. However, it is known that variations uniquely shown in individual genomes may explain individual genetic characteristics related to a phenotypic variation and disease susceptibility, and thus it is very important to find the variations.

However, it is very difficult to generate a sequence, only by using a conventional re-sequencing method, corresponding to a part that does not exist in a reference sequence and is uniquely inserted into an individual genome or a portion that exists in the reference sequence and is shown differently in an individual genome due to factors such as a variation. Also, a problem that information about individual genomes of reads that are not mapped to the reference sequence is lost may not be resolved only by using the conventional re-sequencing method.

### SUMMARY OF THE INVENTION

The present invention provides an apparatus and method for generating a novel sequence in a target genome sequence for generating a novel sequence that does not exist in a reference sequence by using input reads that are not mapped to the reference sequence during genome re-sequencing of a next generation sequencing (hereinafter, referred to as NGS) technology.

According to an aspect of the present invention, there is provided a novel sequence generating apparatus including: a read pair obtaining unit for obtaining read pairs respectively including at least one of unmapped reads that are not mapped to a reference sequence according to a result of re-sequencing for mapping input reads received from a genome sequence sequencer to the reference sequence; a contig generating unit for generating contigs assembled by connecting the unmapped reads of the obtained read pairs; a novel sequence generating unit for generating a novel sequence including at least one contig from among the generated contigs; and a position predicting unit for predicting a position of the generated novel sequence on the reference sequence.

The read pairs may include mapped-unmapped read pairs respectively comprised of a pair of one of mapped reads that are mapped to the reference sequence and one of the unmapped reads, and unmapped-unmapped read pairs respectively comprised of a pair of the unmapped reads.

The contigs may include one or more first contigs assembled by connecting the unmapped reads of the mapped-unmapped read pairs and one or more second contigs assembled by connecting the unmapped reads of the unmapped-unmapped read pairs.

The novel sequence may include a first novel sequence obtained by connecting the first contigs having the same directionality of the mapped reads of the mapped-unmapped read pair, from among the one or more first contigs, and the second contig, and a second novel sequence based on the first contigs having different directionalities of the mapped reads of the mapped-unmapped read pairs.

The novel sequence generating unit may filter the generated contigs based on a mapping quality of the mapped reads of the mapped-unmapped read pairs corresponding to the generated contigs, an average base quality of reads constituting the generated contigs, and lengths of the generated contigs.

The position predicting unit may predict a position of the novel sequence on the reference sequence based on positions of mapped reads on the reference sequence, which are mapped to the reference sequence, from among reads of read pairs used to generate contigs included in the novel sequence.

The novel sequence generating apparatus may further include a type predicting unit for predicting a type of the novel sequence including at least one of a variation novel sequence that exists on the reference sequence but is shown differently from the reference sequence in the target genome sequence reconstituted through the re-sequencing and an insertion novel sequence that is inserted independently from the reference sequence, based on a depth of coverage of reads mapped to the predicted position of the novel sequence on the reference sequence and to a region indicated by the position.

The novel sequence generating apparatus may further include a novel sequence output unit for outputting information regarding the predicted position and the predicted type of the novel sequence.
According to another aspect of the present invention, there is provided a method of generating a novel sequence, the method including: performing re-sequencing for mapping input reads obtained through genome sequence sequencing to a reference sequence; obtaining read pairs respectively including at least one of unmapped reads that are not mapped to the reference sequence according to a result of the re-sequencing; generating contigs assembled by connecting the unmapped reads of the obtained read pairs; generating the novel sequence including at least one contig from among the generated contigs; and predicting a position of the generated novel sequence on the reference sequence.

The obtaining of the read paris may include: obtaining mapped-unmapped read pairs respectively comprised of one of mapped reads mapped to the reference sequence and one of the unmapped reads according to a result of the re-sequencing; and obtaining unmapped-unmapped read pairs respectively comprised of a pair of unmapped reads according to a result of the re-sequencing.

The generating of the contigs may include: generating one or more first contigs assembled by connecting the unmapped reads of the mapped-unmapped read pairs; and generating one or more second contigs assembled by connecting unmapped reads of the unmapped-unmapped read pairs.

The generating of the novel sequence may include: determining whether the one or more first contigs is valid based on mapping positions and directionalities of the mapped reads of the mapped-unmapped read pairs on the reference sequence, which correspond to the first contig; generating a first novel sequence obtained by connecting the first contigs having the same directionality of the mapped reads of the mapped-unmapped read pair, from among the one or more first contigs, and the second contig; and generating a second novel sequence based on the first contigs having different directionalities of the mapped reads of the mapped-unmapped read pairs.

The predicting of the position of the generated novel sequence may include predicting a position of the novel sequence on the reference sequence based on positions of mapped reads on the reference sequence, which are mapped to the reference sequence, from among reads of read pairs used to generate contigs included in the novel sequence.

The method may further include a type predicting unit for predicting a type of the novel sequence based on a depth of coverage of reads mapped to the predicted position of the novel sequence on the reference sequence and to a region indicated by the position, wherein the type of the novel sequence may include at least one of a variation novel sequence that exists on the reference sequence but is shown differently from the reference sequence in the target genome sequence reconstituted through the re-sequencing and an insertion novel sequence that is inserted independently from the reference sequence.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram showing a genome sequence analyzing system, according to an embodiment of the present invention;
FIG. 2 is a block diagram of a novel sequence generating apparatus, according to an embodiment of the present invention;
FIGS. 3A and 3B are diagrams for describing concepts of read pairs and contigs, according to an embodiment of the present invention;
FIG. 4 is a flowchart showing a method of generating a novel sequence and predicting information about the novel sequence, according to an embodiment of the present invention;
FIG. 5A is a flowchart showing a process of generating a novel sequence based on contigs, according to an embodiment of the present invention;
FIG. 5B is a diagram for describing an example of determining whether contigs are valid during generation of a novel sequence, according to an embodiment of the present invention;
FIGS. 6A and 6B are diagrams for describing a process of predicting information about a novel sequence generated according to an embodiment of the present invention;
FIG. 7 is a diagram showing a process of classifying types of contigs by determining whether a first contig is valid, according to an embodiment of the present invention;
FIG. 8 is a pseudo-code showing a process for generating a novel sequence by connecting first contigs having the same directionality of mapped reads of mapped-unmapped read pairs, from among the first contigs, and second contigs, according to an embodiment of the present invention.

### DETAILED DESCRIPTION OF THE INVENTION

The preceding merely illustrates the principles of the invention. It will thus be appreciated that one of ordinary skill in the art will be able to devise various arrangements which, although not explicitly described or shown herein, embody the principles of the invention and are included within its spirit and scope. Furthermore, all examples and conditional language recited herein are principally intended expressly to be only for pedagogical purposes and to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention, as well as specific examples thereof, are intended to encompass both structural and functional equivalents thereof. Additionally, it is intended that such equivalents include both currently known equivalents and equivalents developed in the future, i.e., any elements developed that perform the same function, regardless of structure.

Functions of various devices that are illustrated in drawings including a function block denoted as a processor or as a similar concept with the processor, can be provided not only with specific hardware but also general hardware in which related software may be executed. When these functions are provided by the processor, the functions may be provided by a singular specific processor, a singular sharable processor, or plural processors in which sharing between the plural processors is possible. Also, usage of terms such as a processor, a control, or the like should not be construed as being limited to hardware capable of executing software but should be construed as indirectly including digital signal processor (DSP) hardware, read-only memory (ROM), random-access memory (RAM), and non-volatile memory used for storing software. Other well-known conventional hardware devices may be included.

Hereinafter, the present invention will be described in detail by explaining exemplary embodiments of the invention with reference to the attached drawings.
In the following description of the present invention, only essential parts necessary to understand operation of the present invention will be explained and other parts will not be explained when it is deemed that they make unnecessarily obscure the subject matter of the invention.

Unless noted otherwise, the word "comprise" or variations such as "comprises" or "comprising" is understood to mean "includes, but is not limited to" so that other elements that are not explicitly mentioned may also be included. Expressions such as "at least one of," when preceding a list of elements, modify the entire list of elements and do not modify the individual elements of the list.

The present invention will now be described more fully with reference to the accompanying drawings, in which exemplary embodiments of the invention are shown.

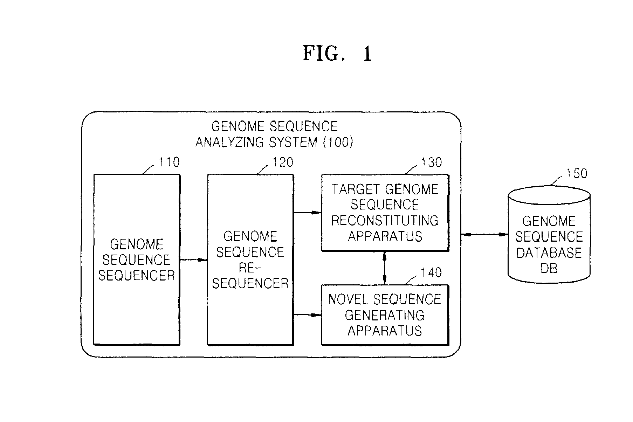

FIG. 1 is a block diagram showing a genome sequence analyzing system 100, according to an embodiment of the present invention.

Referring to FIG. 1, the genome sequence analyzing system 100 may include a genome sequence sequencer 110, a genome sequence re-sequencer 120, a target genome sequence reconstituting apparatus 130, and a novel sequence generating apparatus 140. The genome sequence analyzing system 100 may obtain information regarding a target genome sequence or a reference sequence from a genome sequence database 150 or may generate information regarding a novel sequence and store the information in the genome sequence database 150.

The genome sequence sequencer 110 generates base sequence data of a target genome through sequencing. Although a target life is not limited to a human being, a reference sequence for analyzing a genome should exist.

In the current embodiment, the base sequence data refers to data regarding a sequence of four bases A, C, G, and T constituting deoxyribonucleic acid (DNA) generated using a DNA sequencer, and data attached thereto. Here, the attached data may be, for example, a base quality score and a read depth.

The genome sequence re-sequencer 120 receives input reads constituting the base sequence of the target genome from among the base sequence data from the genome sequence sequencer 110 and performs re-sequencing for mapping the input reads to the reference sequence.

In the current embodiment, the input reads refer to a single connected base read generated through DNA sequencing in the genome sequence sequencer 110. Since division and proliferation of DNA are performed during the DNA sequencing, overlapped portions may exist in the reads produced according to a result of the DNA sequencing.

The target genome sequence reconstituting apparatus 130 reconstitutes the target genome sequence based on mapped reads mapped to the reference sequence through re-sequencing in the genome sequence re-sequencer 120.

The novel sequence generating apparatus 140 generates a novel sequence differently formed from the reference sequence due to insertion or variations based on unmapped reads that are not mapped to the reference sequence through the re-sequencing in the genome sequence re-sequencer 120.

Accordingly, the genome sequence analyzing system 100 may provide information regarding the target genome sequence having a more complete structure by combining information regarding the generated novel sequence and information regarding the reconstituted target genome sequence.

As such, in order to provide the information regarding the target genome sequence having a more complete structure, the current embodiment provides an apparatus and method for analyzing a genome sequence by using not only mapped reads mapped to the reference sequence through re-sequencing but unmapped reads.

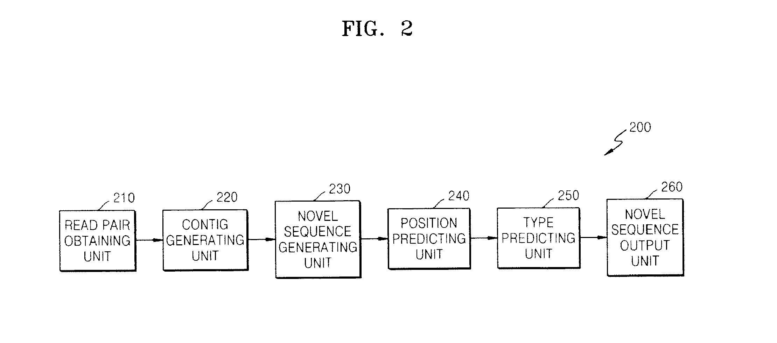

FIG. 2 is a block diagram of a novel sequence generating apparatus 200, according to an embodiment of the present invention.

Referring to FIG. 2, the novel sequence generating apparatus 200 may include a read pair obtaining unit 210, a contig generating unit 220, a novel sequence generating unit 230, a position predicting unit 240, a type predicting unit 250, and a novel sequence output unit 260.

The read pair obtaining unit 210 obtains read pairs respectively including at least one of unmapped reads that are not mapped to the reference sequence according to a result of the re-sequencing for mapping the input reads received from the genome sequence sequencer 110 to the reference sequence.

The read pair obtaining unit 210 is subject to use paired read information provided from a mate-pair library or a paired-end library.

The read pairs may be classified with mapped-mapped read pairs comprised of mapped read pairs mapped to the reference sequence, mapped-unmapped read pairs comprised of mapped reads and unmapped sequence, and unmapped-unmapped read pairs comprised of unmapped read pairs. However, from among these, the read pair obtaining unit 210 may obtain read pairs including at least one of unmapped reads that are not mapped to the reference sequence, that is, the mapped-unmapped read pairs and the unmapped-unmapped read pairs.

The contig generating unit 220 generates assembled contigs by connecting the unmapped reads of the read pairs obtained by the read pair obtaining unit 210.

A representative method of generating a contig may be, for example, a de novo assembly algorithm. In general, the de novo assembly algorithm such as Velvet (Zebrano and Birney, Velvet: algorithms for de novo short read assembly using de Bruijn graphs, Genome research, 18:821-829, 2008), ABYSS (Simpson et al., ABYSS: a parallel assembler for short read sequence data, Genome research, 19:1117-1123, 2009), or SOAPdenovo (Li et al., De novo assembly of human genomes with massively parallel short read sequencing, Genome research, 20:265-272, 2010) is widely used, but the present invention does not limit an algorithm connecting unmapped reads.

Most de novo assembly algorithms require a large capacity of memory according to a size of data to be input. Thus, in order to minimize memory resources consumed during a process of generating the contigs, the contig generating unit 220 may perform de novo assembling according to chromosomes among unmapped reads of the read pairs including the mapped reads mapped to the same chromosome sequence.

The contigs generated by the contig generating unit 220 may be classified according to types of the read pairs forming the basis of each of assemblies of the contigs, that is, according to which one of the mapped-unmapped read pairs or the unmapped-unmapped read pairs the contigs correspond to.

In the current embodiment, the contigs assembled by connecting the unmapped reads of the mapped-unmapped read pairs is referred to as 'first contigs', and the contigs assembled by connecting the unmapped reads of the unmapped-unmapped read pairs is referred to as 'second contigs'.

The novel sequence generating unit 230 generates a novel sequence including at least one valid contig from among the contigs generated by the contig generating unit 220.

The novel sequence generating unit 230 may filter invalid contigs from among the contigs generated by the contig generating unit 220 based on a mapping quality of the mapped reads of the corresponding mapped-unmapped read pairs, an average base quality of the reads constituting the contigs, and lengths of the contigs.

For example, in the contigs with reads having a low mapping quality or base quality, since it is difficult to rely on the contigs even though the reads are mapped to the reference sequence, the contigs may be regarded as invalid contigs and may be filtered to obtain a more reliable result.

The novel sequence generating unit 230 may differently process the first contigs generated by the contig generating unit 220 in a case where the mapped reads of the corresponding mapped-unmapped read pairs have the same directionality and a case where the mapped reads of the corresponding mapped-unmapped read pairs have different directionalities.

For example, the first contigs having the same directionality of the mapped reads of the corresponding mapped-unmapped read pairs may be connected to the second contigs to generate the novel sequence.

Also, the novel sequence may be generated based on only the first contigs having the different directionalities of the mapped reads of the corresponding mapped-unmapped read pairs.

The position predicting unit 240 predicts a position of the novel sequence generated by the novel sequence generating unit 230 on the reference sequence. The position predicting unit 240 searches whether mapped reads mapped to the reference sequence exist from among the reads of the read pairs used to generate the contigs included in the novel sequence. If the mapped reads mapped to the reference sequence exist, the position predicting unit 240 may predict a position of a heading novel sequence in the reference sequence based on positions of the mapped reads on the reference sequence.

The type predicting unit 250 may predict a type of the novel sequence based on the position of the novel sequence predicted by the position predicting unit 240 on the reference sequence.

In the current embodiment, the types of the novel sequence may include a variation novel sequence that exists on the reference sequence but is shown differently from the reference sequence in the target genome sequence reconstituted through the re-sequencing, and an insertion novel sequence that is inserted independently from the reference sequence.

The novel sequence output unit 260 outputs information regarding the position of the novel sequence predicted by the position predicting unit 240 and the type predicted by the type predicting unit 250 and information regarding the novel sequence. The novel sequence output unit 260 may provide database for managing genome sequence information and the information regarding the novel sequence to a terminal providing the genome sequence information via a display device.

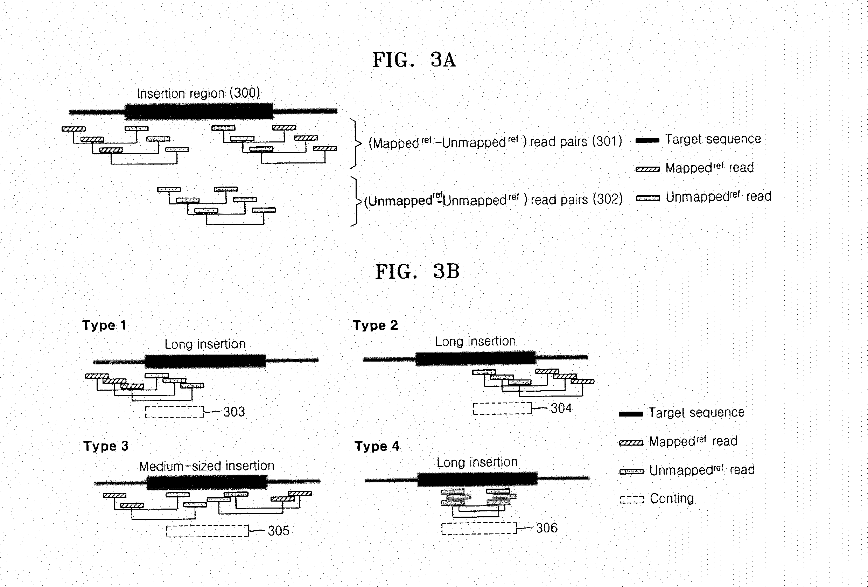

FIG. 3A is a diagram for describing a concept of the read pairs obtained by the novel sequence generating apparatus 200, according to an embodiment of the present invention.

Referring to FIG. 3A, in the reads constituting the novel sequence generated due to insertion, the reads corresponding to an insertion region 300 are not mapped to the reference sequence according to a result of the re-sequencing.

Accordingly, in order to generate (restore) the novel sequence having the reads that are not mapped to the reference sequence according to a result of the re-sequencing, the novel sequence generating apparatus obtains, from among results of re-sequencing of a genome sequence input to the genome sequence analyzing system 100, (1) read pairs (hereinafter, referred to as mapped-unmapped read pairs or Mapped^{ref}-Unmapped^{ref} read pairs) 301 in which one read is mapped to the reference sequence (hereinafter, referred to a mapped read or a Mapped^{ref} read), but the other one read is not mapped to the reference sequence (hereinafter, referred to an unmapped read or an Unmapped^{ref} read) and (2) read pairs (hereinafter, referred to as unmapped-unmapped read pairs or Unmapped^{ref}-Unmapped^{ref} read pairs) 302 in which both the reads are not mapped to the reference sequence.

FIG. 3B is a diagram for describing a concept of a contig generated by the novel sequence generating apparatus 200, according to an embodiment of the present invention.

In the current embodiment, when a novel sequence which is midium in length, that is, a novel sequence of which entire length is less than twice an insert size between reads forming a pair, the novel sequence may be generated (restored) by using only a contig 305 assembled by connecting unmapped reads of the mapped-unmapped read pairs (see Type 3). However, when a novel sequence which is long in length, that is, a novel sequence of which entire length is equal to or greater than twice the insert size between the read pairs, the novel sequence may not be generated (restored) outside of genome sequences corresponding to both ends of the novel sequence by using only contigs 303 and 304 assembled by connecting the unmapped reads of the mapped-unmapped read pairs (see Type 1 and Type 2). Accordingly, in the novel genome sequence which is long in length, the entire novel sequence may be generated (restored) only when the contigs 303 and 304 are connected to a contig 306 (see Type 4) assembled by connecting the unmapped reads of the unmapped-unmapped read pairs.

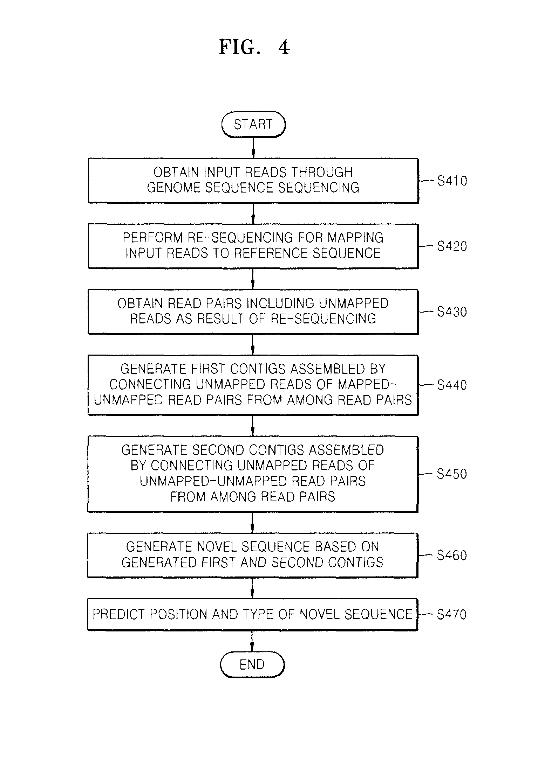

FIG. 4 is a flowchart showing a method of generating a novel sequence and predicting information about the novel sequence, according to an embodiment of the present invention. The method of generating the novel sequence may be performed by the genome sequence analyzing system 100 shown in FIG. 2 and the novel sequence generating apparatus 200 shown in FIG. 2. Thus, a repeated description with regard to the genome sequence analyzing system 100 shown in FIG. 2 and the novel sequence generating apparatus 200 shown in FIG. 2 will be omitted.

Referring to FIG. 4, first, the input reads are obtained through genome sequence sequencing (operation S410).

Re-sequencing for mapping the input reads obtained in operation S410 to the reference sequence is performed (operation S420).

The read pairs respectively including at least one of the unmapped reads that are not mapped to the reference sequence according to a result of the re-sequencing in operation S420, that is, the mapped-unmapped read pairs and the unmapped-unmapped read pairs are obtained (operation S430).

The first contigs assembled by connecting the unmapped reads of the mapped-unmapped read pairs from among the read pairs obtained in operation S430 are generated (operation S440), and the second contigs assembled by connecting the unmapped reads of the unmapped-unmapped read pairs from among the read pairs obtained in operation S430 are generated (operation S450).

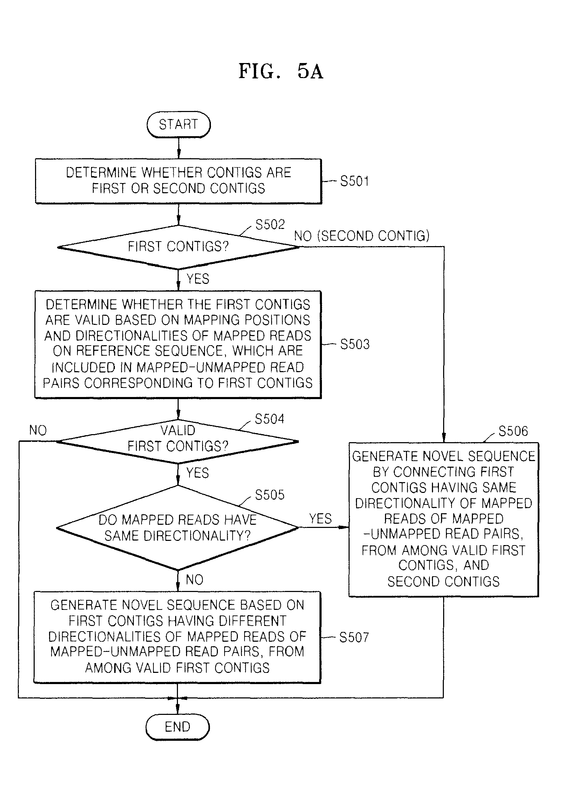

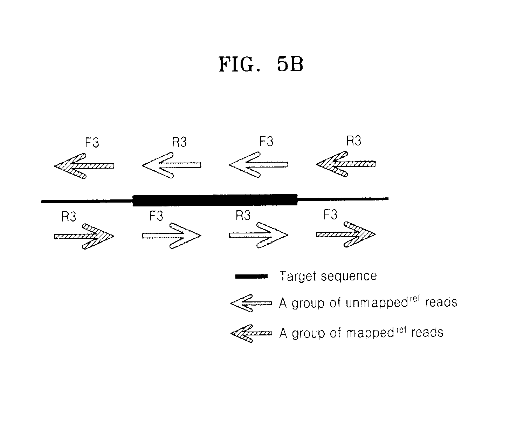

The novel sequence is generated based on the first and second contigs generated in operations S440 and S450 (operation S460). A detailed example of generating the novel sequence based on the contigs in operation S460 will be described with reference to FIGS. 5A and 5B.

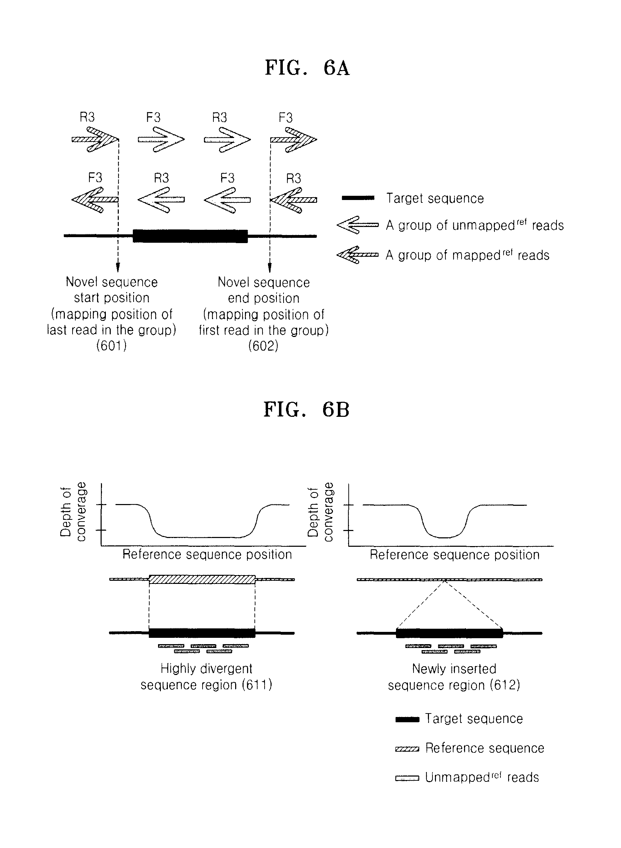

The position and type of the novel sequence generated in operation S460 are predicted (operation S470). Here, the position of the novel sequence on the reference sequence may be predicted based on the position of the mapped reads on the reference sequence, which are mapped to the reference sequence, from among the reads of the read pairs used to generate the contigs included in the novel sequence. In the current embodiment, a detailed example of predicting the position and type of the novel sequence will be described with reference to FIG. 6.

FIG. 5A is a flowchart showing a process of generating the novel sequence based on contigs, according to an embodiment of the present invention. FIG. 5B is a diagram for describing an example of determining whether contigs are valid during generation of the novel sequence, according to an embodiment of the present invention.

Referring to FIG. 5A, it is determined whether the contigs are the first or second contigs (operation S501).

According to a result of the determination in operation S501, when the contigs are the first contigs (operation S502), it is determined whether the first contigs are valid based on mapping positions and directionalities of the mapped reads on the reference sequence, which are included in the mapped-unmapped read pairs corresponding to the first contigs (operation S503).

The determining of whether the first contigs are valid in operation S503 is performed to filter random contigs not related to the novel sequence. Since the first contigs are generated by using the unmapped reads of the mapped-unmapped read pairs, the mapping positions and directionalities of the mapped reads on the reference sequence, which form pairs with the corresponding unmapped reads for the filtering, may be considered.

For example, if the mapping positions of the mapped reads are closely-disposed within a predetermined distance and the mapped reads have the same directionality, it may be determined that the corresponding contigs are valid, and the contigs may be determined to be the Type 1 contigs 303 (see FIG. 3B) or the Type 2 contigs 304 (see FIG. 3B) according to the directionalities of the mapped reads.

Also, although the mapped reads have different directionalities, if the positions of the mapped reads having the same directionality are within a predetermined distance and if a group of two reads having the same directionality, that is, a group of the mapped reads and a group of the unmapped reads do not overlap with each other, it may be determined that the corresponding contigs are valid, and thus it may be determined that the corresponding contigs are Type 3 contigs 305 (see FIG. 3B).

As such, according to a result of the determination of whether the contigs are valid in consideration of the mapping positions and directionalities of the mapped reads on the reference sequence, invalid contigs are determined to be meaningless random contigs, and thus the invalid contigs are excluded (filtered) during the generation of the novel sequence (operation S504).

Then, it is determined with respect to the first contigs determined to be valid in operation S503 whether the mapped reads of the mapped-unmapped read pairs have the same directionality (operations S504 and S505). If the first contigs have the same directionality, the novel sequence is generated by connecting the first contigs and the second contigs (operation S506).

As described above, the first contigs having the same directionality of the mapped reads of the mapped-unmapped read pairs may be classified into the Type 1 contigs 303 and the Type 2 contigs 304 (see FIG. 3B), and the Type 1 contigs 303 and the Type 2 contigs 304 are connected to the Type 4 contig 306, that is, the second contig (see FIG. 3B) to generate a contig (novel sequence) which is long in length.

Here, when a sequence of a suffix of the Type 1 contig 303 overlaps with a sequence of a prefix of the Type 4 contig 306 or when a sequence of a prefix of the Type 2 contig 304 overlaps with a sequence of a suffix of the Type 4 contig 306, the sequences may be connected to one another. In other words, when the sequences are connected to one another in the order of Type 1 > Type 4 > Type 2, or the sequences overlap with one another in the order of Type 1 > Type 4 or Type 4 > Type 2, the sequences may be connected to one another to generate a single long contig (novel sequence).

From among the first contigs determined to be valid in operation S502, the novel sequence is generated based on the first contigs having different directionalities of the mapped reads of the mapped-unmapped read pairs (operation S507).

As described above, the valid first contigs having different directionalities of the mapped reads of the mapped-unmapped read pairs may be classified as the Type 3 contig 305 (see FIG. 3B), and the Type 3 contig 305 may be a contig (novel sequence) which is midium in length.

The novel sequence generated in operation S506 or S507 may correspond to a medium-sized novel sequence and a long novel sequence, or one of the medium-sized novel sequence and the long novel sequence. Also, the target genome sequence having a more complete structure may be provided by providing information about the novel sequence.

FIGS. 6A and 6B are diagrams for describing a process of predicting information about the novel sequence generated according to an embodiment of the present invention.

In the current embodiment, the information about the novel sequence, that is, the position of the novel sequence on the reference sequence, may be predicted based on the potions of the mapped reads on the reference sequence from among the reads of the read pairs used to generate the contigs included in the novel sequence.

Referring to FIG. 6A, a contig formed by connecting the Type 3 contig corresponding to the novel sequence which is midium in length and the Types 1, 2, and 4 contigs may predict a start position 601 and an end position 602 of the novel sequence on the reference sequence.

However, the novel sequence corresponding to the contig formed by connecting the Type 1 contig and the Type 4 contig may predict only the start position 601, and the novel sequence corresponding to the contig formed by connecting the Type 4 contig and the Type 2 contig may predict only the end position 602. Here, the predicted position of the novel sequence on the reference sequence may mean that an insertion event occurs in a region indicated by the corresponding position of the reference sequence or that highly divergent sequence exists in the region indicated by the corresponding position of the reference sequence.

Also, the type of the novel sequence may be predicted based on a depth of coverage of the mapped reads mapped to the predicted position of the novel sequence on the reference sequence or to the region indicated by the corresponding position of the reference sequence due to the fact that since the region including the novel sequence generally have a less number of mapped reads than a peripheral region, the depth of coverage of the corresponding region is far less than an average depth of coverage.

A method of determining a type of a novel sequence, which is to be described below, is performed by using a copy number variation (CNV) algorithm using a depth of coverage. The current embodiment will be described by using a part of a CNVnator algorithm (Abyzov et al., CNVnator: an approach to discover, geno type, and characterize typical and atypical CNVs from family and population genome sequencing, Genome research 21:974-984, 2011). However, this is just an example for ease of description, and the present invention is not limited thereto.

A region which includes front and rear areas within a predetermined distance on the reference sequence and is predicted to have the novel sequence is set to a target region, and the target region is divided into small bins having a predetermined size to calculate a dept of coverage of the mapped reads. As shown in the CNVnator algorithm, the depth of coverage may be adjusted by considering a correlation between the depth of coverage and a GC content. Also, the target region is divided into segments showing depths of coverage having different patterns by using a partitioning algorithm

According to the current embodiment, since a single novel sequence exists in the target region, the target region may be divided into a novel sequence region and front and rear neighboring regions. Since the reads may not be or may not be easily mapped to the novel sequence region compared to the neighboring regions, and thus the novel sequence region may have a lower depth of coverage than the neighboring regions. If the novel sequence region having a lower depth of coverage have a length similar to or longer than that of the corresponding contig of the predicted novel sequence, the novel sequence of the target region may be determined to be a highly divergent sequence type (hereinafter, referred to as a divergent novel sequence), or if the novel sequence region has a length shorter than that of the corresponding contig of the predicted novel sequence, the novel sequence of the target region may be determined to be an insertion generation type (hereinafter, referred to as an insertion novel sequence).

For example, referring to FIG. 6B, in a region 611 where the highly divergent sequence exists, a region having a low depth of coverage may be distributed in correspondence to a length of the novel sequence.

Meanwhile, in a region 612 where the insertion event occurs, since the corresponding novel sequence is inserted into a specific break point in a predicted region, a region having a low depth of coverage may be shown a significantly narrow region, or may not be easily distinguished.

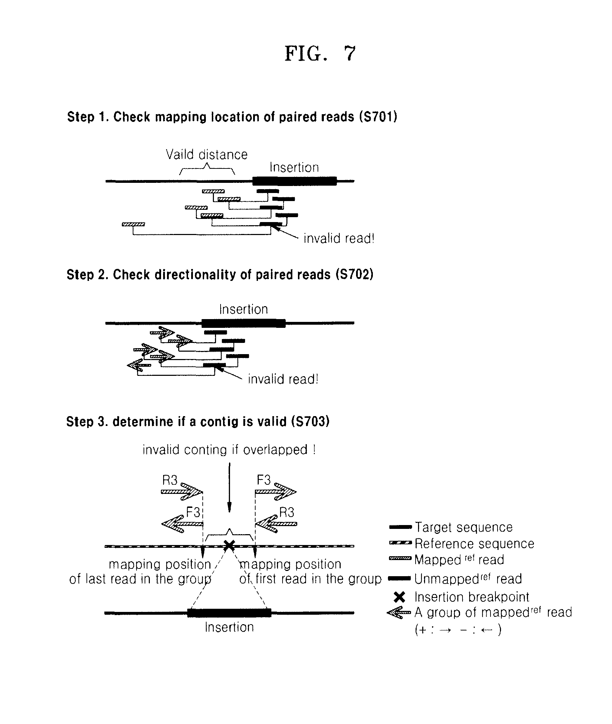

FIG. 7 is a diagram showing a process of classifying types of the contigs by determining whether the first contig is valid, according to an embodiment of the present invention.

Referring to FIG. 7, from among the generated contigs, each of the first contigs (the Types 1, 2, and 3) may be filtered in consideration of the mapping positions and directionalities of the mapped reads on the reference sequence, which form pairs with the unmapped reads used to generate the contigs. In the current embodiment, although the mate-pair library of a SOLiD sequencer is used, this is just an example for ease of description, and the present invention is not limited thereto. For the filtering of the contigs, first, validity of the unmapped reads used to generate each of the first contigs is examined. If the unmapped reads are valid, the mapping positions of the mapped reads forming pairs should be adjacent to the positions of the mapped reads forming pairs with other unmapped reads. Otherwise, the unmapped reads are determined to be invalid, and thus the contigs may be filtered (operation S701).

Also, F3 or R3 mapped reads forming pairs should have the same strand (+ or -). Otherwise, the reads are determined to be invalid, and thus the contigs may be filtered (operation S702). If each of the contigs includes the invalid unmapped reads at a predetermined ratio or more, the contig is determined to be invalid, and thus the contig may be filtered.

In addition, when the type of the first contig is classified at the same time when the contig is filtered, if all the mapped reads forming pairs with the valid unmapped reads of the first contig are F3 mapped reads, the first contig may be classified as the Type 2 contig if the F3 mapped reads are + strands, and the first contig may be classified as the Type 1 contig if the F3 mapped reads are - strands.

Meanwhile, if all the mapped reads forming pairs with the valid unmapped reads of the first contig are R3 mapped reads, the first contig may be classified as the Type 1 contig if the R3 mapped reads are + strands, and the first contig may be classified as the Type 2 contig if the R3 mapped reads are - strands.

Also, even though the mapped reads forming pairs with the valid unmapped reads of the first contig are a mixture of the F3 and R3 mapped reads, if the F3 and R3 mapped reads are different types of strands, the first contig may be the Type 1 or Type 2 contig.

If the mapped reads forming pairs with the valid unmapped reads of the first contig are a mixture of the F3 and R3 mapped reads and if the F3 and R3 mapped reads are the same type of strand, validity and types of the mapped reads may be determined in consideration of mapped regions of the F3 reads and the R3 reads (operation S703). If the and R3 mapped reads are + strands, the mapped region of the R3 reads should be disposed in front of the mapped region of the F3 reads. On the contrary, if the and R3 mapped reads are - strands, the mapped region of the F3 reads should be disposed in front of the mapped region of the R3 reads. If the conditions are satisfied, the first contig may be classified as the Type 3 contig, or otherwise, the first contig is determined to be an invalid contig, and thus the first contig may be filtered.

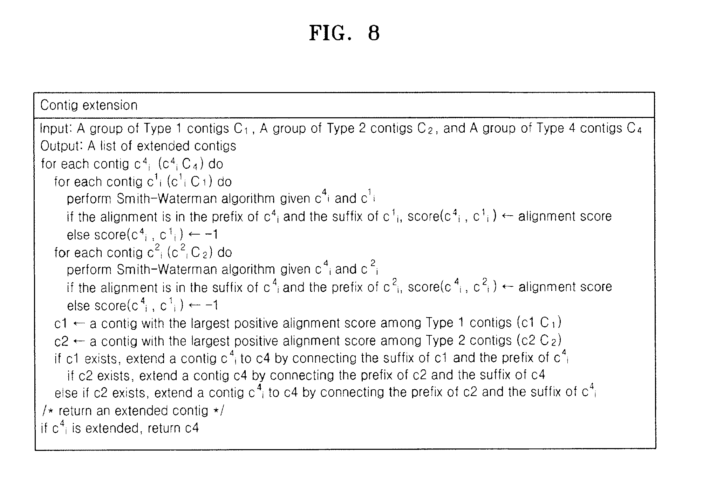

FIG. 8 is a pseudo-code showing a process for generating a novel sequence by connecting the first contigs having the same directionality of the mapped reads of the mapped-unmapped read pairs, from among the first contigs, and the second contigs, according to an embodiment of the present invention.

According to the current embodiment, the second contigs (Type 4 contigs) may be connected to the first contigs (Type 1 contigs and Type 2 contigs) having the same directionality of the mapped reads of the mapped-unmapped read pairs, from among the first contigs, and thus the contigs may be extended.

As such, to connect the contigs, a sequence of a suffix of the Type 1 contig should overlap with a sequence of a prefix of the Type 4 contig, or a sequence of a prefix of the Type 1 contig should overlap with a sequence of a suffix of the Type 4 contig.

In order to information regarding overlapping between the sequences of the contigs, the current embodiment uses a Smith-Waterman algorithm (Smith and Waterman, Identification of common molecular subsequences, J. Mol. Biol., 147:195-197, 1981) that calculates an optimal local alignment between the two sequences. However, this is just an example for ease of description, and the present invention is not limited thereto.

As described in the current embodiment, in order to connect the contigs, first, an alignment between the Type 4 contig and the Types 1 and 2 is calculated, and it is determined whether the alignment is located in a region where the sequence of the Type 4 contig exists. If an alignment exists between the sequence of one Type 4 contig and the sequence of at least one Type 1 or 2 contig, the Type 1 or 2 contig having a largest alignment score may be used for connection of the Type 4 contig.

Also, the Type 1 or 2 contig that is not used for the extension of the contig, as well as the contig extended to provide more information regarding the novel sequence in the target genome sequence, may be realized to be reported as a partial sequence belonging to the novel sequence.

According to the present invention, a novel sequence that is not reflected to a reference sequence of a target genome sequence is generated, and information regarding the novel sequence may be provided. Also, extensive research into individual genetic characteristics may be conducted based on the information regarding the novel sequence and conventional NGS data. In addition, the target genome sequence having a more complete structure may be provided by combining information regarding the target genome sequence reconstituted through re-sequencing and information regarding the novel sequence generated according to the present invention. Eventually, more detailed information regarding individual genetic variations may be obtained, and this may contribute to development of research into a customized genome sequence.

The present invention may be embodied as computer-readable codes in a computer-readable recording medium. The computer-readable recording medium may be any recording apparatus capable of storing data that is read by a computer system. Examples of the computer-readable recording medium include read-only memories (ROMs), random-access memories (RAMs), CD-ROMs, magnetic tapes, floppy disks, and optical data storage devices. The computer-readable recording medium may be a carrier wave that transmits data via the Internet, for example. The computer readable medium may be distributed among computer systems that are interconnected through a network, and the present invention may be stored and implemented as computer readable codes in the distributed system. Functional programs, codes, and code segments for embodying the present invention may be easily derived by programmers in the technical field to which the present invention pertains.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A novel sequence generating apparatus comprising:
a read pair obtaining unit for obtaining read pairs respectively comprising at least one of unmapped reads that are not mapped to a reference sequence according to a result of re-sequencing for mapping input reads received from a genome sequence sequencer to the reference sequence;
a contig generating unit for generating contigs assembled by connecting the unmapped reads of the obtained read pairs;
a novel sequence generating unit for generating a novel sequence comprising at least one contig from among the generated contigs; and
a position predicting unit for predicting a position of the generated novel sequence on the reference sequence.

2. The novel sequence generating apparatus of claim 1, wherein the read pairs comprise mapped-unmapped read pairs respectively comprised of a pair of one of mapped reads that are mapped to the reference sequence and one of the unmapped reads, and unmapped-unmapped read pairs respectively comprised of a pair of the unmapped reads.

3. The novel sequence generating apparatus of claim 2, wherein the contigs comprise one or more first contigs assembled by connecting the unmapped reads of the mapped-unmapped read pairs and one or more second contigs assembled by connecting the unmapped reads of the unmapped-unmapped read pairs.

4. The novel sequence generating apparatus of claim 3, wherein the novel sequence comprises a first novel sequence obtained by connecting the first contigs having the same directionality of the mapped reads of the mapped-unmapped read pair, from among the one or more first contigs, and the second contig, and a second novel sequence based on the first contigs having different directionalities of the mapped reads of the mapped-unmapped read pairs.

5. The novel sequence generating apparatus of any one of claims 1 to 4, wherein the novel sequence generating unit filters the generated contigs based on a mapping quality of the mapped reads of the mapped-unmapped read pairs corresponding to the generated contigs, an average base quality of reads constituting the generated contigs, and lengths of the generated contigs.

6. The novel sequence generating apparatus of any one of claims 1 to 5, wherein the position predicting unit predicts a position of the novel sequence on the reference sequence based on positions of mapped reads on the reference sequence, which are mapped to the reference sequence, from among reads of read pairs used to generate contigs comprised in the novel sequence.

7. The novel sequence generating apparatus of any one of claims 1 to 6, further comprising a type predicting unit for predicting a type of the novel sequence comprising at least one of a variation novel sequence that exists on the reference sequence but is shown differently from the reference sequence in the target genome sequence reconstituted through the re-sequencing and an insertion novel sequence that is inserted independently from the reference sequence, based on a depth of coverage of reads mapped to the predicted position of the novel sequence on the reference sequence and to a region indicated by the position.

8. The novel sequence generating apparatus of claim 7, further comprising a novel sequence output unit for outputting information regarding the predicted position and the predicted type of the novel sequence.

9. A method of generating a novel sequence, the method comprising:
performing re-sequencing for mapping input reads obtained through genome sequence sequencing to a reference sequence;
obtaining read pairs respectively comprising at least one of unmapped reads that are not mapped to the reference sequence according to a result of the re-sequencing;
generating contigs assembled by connecting the unmapped reads of the obtained read pairs;
generating the novel sequence comprising at least one contig from among the generated contigs; and
predicting a position of the generated novel sequence on the reference sequence.

10. The method of claim 9, wherein the obtaining of the read pairs comprises:
obtaining mapped-unmapped read pairs respectively comprised of one of mapped reads mapped to the reference sequence and one of the unmapped reads according to a result of the re-sequencing; and
obtaining unmapped-unmapped read pairs respectively comprised of a pair of unmapped reads according to a result of the re-sequencing.

11. The method of claim 9 or 10, wherein the generating of the contigs comprises:
generating one or more first contigs assembled by connecting the unmapped reads of the mapped-unmapped read pairs; and
generating one or more second contigs assembled by connecting unmapped reads of the unmapped-unmapped read pairs.

12. The method of claim 11, wherein the generating of the novel sequence comprises:
determining whether the one or more first contigs is valid based on mapping positions and directionalities of the mapped reads of the mapped-unmapped read pairs on the reference sequence, which correspond to the first contig;
generating a first novel sequence obtained by connecting the first contigs having the same directionality of the mapped reads of the mapped-unmapped read pair, from among the one or more first contigs, and the second contig; and
generating a second novel sequence based on the first contigs having different directionalities of the mapped reads of the mapped-unmapped read pairs.

13. The method of any one of claims 9 to 12, wherein the predicting of the position of the generated novel sequence comprises predicting a position of the novel sequence on the reference sequence based on positions of mapped reads on the reference sequence, which are mapped to the reference sequence, from among reads of read pairs used to generate contigs comprised in the novel sequence.

14. The method of any one of claims 9 to 13, further comprising a type predicting unit for predicting a type of the novel sequence based on a depth of coverage of reads mapped to the predicted position of the novel sequence on the reference sequence and to a region indicated by the position,
wherein the type of the novel sequence comprises at least one of a variation novel sequence that exists on the reference sequence but is shown differently from the reference sequence in the target genome sequence reconstituted through the re-sequencing and an insertion novel sequence that is inserted independently from the reference sequence.
